# EUROPÄISCHE PATENTSCHRIFT

(11) **EP 4 596 013 B1**
(45) Veröffentlichungstag und Bekanntmachung des Hinweises auf die Patenterteilung: **15.07.2026**
(21) Anmeldenummer: 25154454.0
(22) Anmeldetag: 28.01.2025
(51) Int. Cl.: A61M 16/00, A61M 16/06, A61M 16/08

(54) **NASENKANÜLE ZUM VERSORGEN EINES PATIENTEN MIT ATEMGAS IM RAHMEN EINER HIGH-FLOW-THERAPIE**
NASAL CANNULA FOR SUPPLYING A PATIENT WITH BREATHING GAS IN THE CONTEXT OF HIGH-FLOW THERAPY
CANULE NASALE POUR FOURNIR UN GAZ RESPIRATOIRE À UN PATIENT À UN TRAITEMENT À HAUT DÉBIT

(30) Priorität: 01.02.2024 DE 102024102820
(43) Veröffentlichungstag der Anmeldung: 06.08.2025
(73) Patentinhaber: Löwenstein Medical Technology S.A., 2557 Luxembourg (LU)
(72) Erfinder: Specht, Andreas, 56283 Pfaffenheck (DE)
(74) Vertreter: Löwenstein Medical IP

(56) Entgegenhaltungen:
- WO-A1-2011/110961
- WO-A1-2015/020540
- WO-A1-2016/043607
- WO-A1-2020/178686
- WO-A2-2014/182179

## Beschreibung

### Technisches Gebiet

Die Erfindung betrifft eine Nasenkanüle zum Versorgen eines Patienten mit Atemgas im Rahmen einer High-Flow-Therapie. Des Weiteren betrifft die Erfindung ein Beatmungssystem mit einer solchen Nasenkanüle.

### Stand der Technik

Die nasale High-Flow-Therapie mittels einer Nasenkanüle kann beispielsweise bei Patienten mit leichtem bis mittelschwerem Atemnotsyndrom mit akuter hypoxämischer respiratorischer Insuffizienz angewandt werden. Dabei kann durch einen kontinuierlichen Frischgasfluss der anatomische Totraum, d. h. das Volumen der Atemwege von der Nasenhöhle bis hin zu den Endbrochiolen, die nicht am Gasaustausch teilnehmen, als Sauerstoffreservoir genutzt und somit funktionell verkleinert werden. Die nasale High-Flow-Therapie kann das Atemzugvolumen erhöhen, während die Atemfrequenz sinkt. Auf diese Weise kann die alveoläre Ventilation verbessert und die Atemarbeit unterstützt werden.

Die nasale High-Flow-Therapie wird zunehmend bei sehr kleinen Patienten wie Früh- oder Neugeborenen, Säuglingen oder Babys eingesetzt, wo sie den Therapieerfolg im Vergleich zu einer konventionellen Sauerstofftherapie spürbar verbessern kann. Bei der nasalen High-Flow-Therapie mit diesen Patienten liegt der Sauerstoffdurchfluss in der Regel zwischen 1 bis 8 I/min.

Eine entsprechende Nasenkanüle (auch Nasenbrille genannt) umfasst in der Regel ein Nasenansatzstück mit zwei Nasenstutzen (auch Prongs genannt) zum Einführen in die Nasenlöcher. Das Nasenansatzstück kann über einen Zufuhrschlauch mit einer Atemgasquelle verbunden werden, sodass Atemgas mit einem geeigneten Durchfluss ins Nasenansatzstück und von dort über die Nasenstutzen weiter in die Nase strömen kann.

Insbesondere bei Früh- oder Neugeborenen kann das Anbringen bzw. Entfernen der Nasenkanüle aufgrund des kleinen Kopfes und der besonders empfindlichen Haut mit gewissen Schwierigkeiten verbunden sein.

Ferner sind bei der nasalen High-Flow-Therapie mangels einer entsprechenden Druckmessung oft mehrere Versuche nötig, bis eine geeignete Einstellung der Beatmungsparameter gefunden wird.

WO 2014/182179 A2 offenbart eine für eine High-Flow-Therapie in der Pädiatrie geeignete Nasenkanüle mit einem Halteband und daran angeordneten Wangenpolstern. Das Halteband kann über Steckverbinder an Befestigungsflügeln eines Nasenansatzstücks befestigt werden.

### Offenbarung der Erfindung

Eine Aufgabe der Erfindung kann darin gesehen werden, eine verbesserte Nasenkanüle zum Versorgen eines Patienten mit Atemgas im Rahmen einer High-Flow-Therapie bereitzustellen. Eine weitere Aufgabe der Erfindung kann darin gesehen werden, ein entsprechendes Beatmungssystem bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Offenbarung betrifft eine Nasenkanüle zum Versorgen eines Patienten in Gestalt eines Früh- oder Neugeborenen, eines Säuglings oder eines Babys mit Atemgas im Rahmen einer (nasalen) High-Flow-Therapie. Die Nasenkanüle umfasst ein Nasenansatzstück mit einem hohlen Grundkörper, einem Schlauchanschluss zum Anschließen eines Zufuhrschlauchs an den Grundkörper, sodass Atemgas aus dem Zufuhrschlauch in den Grundkörper strömen kann, und zwei vom Grundkörper abstehenden Nasenstutzen zum Leiten des Atemgases aus dem Grundkörper in die Nase des Patienten. Ferner umfasst die Nasenkanüle ein erstes Wangenpolster zum Abstützen und/oder Fixieren des Nasenansatzstücks an einer ersten Wange des Patienten, ein zweites Wangenpolster zum Abstützen und/oder Fixieren des Nasenansatzstücks an einer zweiten Wange des Patienten und ein um den Kopf des Patienten spannbares Halteband zum Aufbringen einer zusätzlichen Haltekraft auf das Nasenansatzstück und/oder die beiden (oder mindestens eines der) Wangenpolster.

Die zusätzliche Haltekraft kann geeignet sein, das Nasenansatzstück beim Gebrauch der Nasenkanüle in einer definierten Position gegenüber dem Gesicht des Patienten zu halten.

Die Verwendung eines speziellen Haltebands ermöglicht eine besonders sichere Fixierung der Nasenkanüle an dem sehr kleinen Gesicht des Patienten, auch wenn dieser sehr lebhaft ist.

Unter "Nasenstutzen" kann ein sogenannter Prong zum Einführen in ein einzelnes Nasenloch verstanden werden.

Unter "Halteband" kann ein vom Zufuhrschlauch getrenntes längliches Element wie beispielsweise ein Gummi- und/oder Stoffband oder eine Anordnung aus mehreren solcher Bänder verstanden werden.

Unter "Wangenpolster" kann ein flächiges, besonders biegsames Element aus einem luftdurchlässigen und/oder flüssigkeitsabsorbierenden Material, beispielsweise einem besonders hautfreundlichen und/oder besonders weichen Textilmaterial, verstanden werden. Beim Gebrauch der Nasenkanüle können die Wangenpolster die Haut des Patienten unmittelbar flächig berühren, sodass der Anpressdruck, der durch die zusätzliche Haltekraft, d. h. durch das um den Kopf gespannte Halteband, erzeugt wird, über eine möglichst große Fläche des Gesichts, insbesondere der Wangen, gleichmäßig verteilt wird. Somit können unerwünschte Druckstellen im Gesicht vermieden werden.

Alternativ oder zusätzlich kann die Nasenkanüle je nach Ausführungsform für mindestens einen der folgenden Patiententypen geeignet sein: Kinder, Jugendliche, Erwachsene.

Offenbart wird zudem eine (nicht beanspruchte) Nasenkanüle zum Versorgen eines Patienten mit Atemgas im Rahmen einer (nasalen) High-Flow-Therapie. Die Nasenkanüle umfasst ein Nasenansatzstück mit einem hohlen Grundkörper, einem Schlauchanschluss zum Anschließen eines Zufuhrschlauchs an den Grundkörper, sodass Atemgas aus dem Zufuhrschlauch in den Grundkörper strömen kann, und zwei vom Grundkörper abstehenden Nasenstutzen zum Leiten des Atemgases aus dem Grundkörper in die Nase des Patienten. Ferner umfasst die Nasenkanüle einen Druckmessanschluss zum Anschließen eines Druckmessgeräts an die Nasenkanüle und eine Druckmessleitung, die mit dem Druckmessanschluss fluidisch gekoppelt ist und sich ausgehend vom Druckmessanschluss durch den Grundkörper bis in mindestens einen der Nasenstutzen erstreckt, um eine Druckmessung in der Nase, beispielsweise am Austritt des Nasenstutzens oder der Nasenstutzen, zu ermöglichen.

Eine solche Nasenkanüle ermöglicht eine sehr genaue oropharyngeale Druckmessung während der High-Flow-Therapie. Somit können Beatmungsparameter der High-Flow-Therapie - wie beispielsweise Atemgasdruck, Atemgasfluss, Atemgasvolumen oder Atemgaszusammensetzung - abhängig vom gemessenen Druck sehr präzise gesteuert und/oder ausgewertet werden. Dies kann die High-Flow-Therapie im Vergleich zu Ausführungsformen ohne eine derartige direkte Messung des Drucks in der Nase deutlich verbessern. Die Nasenkanüle kann je nach Ausführungsform für mindestens einen der folgenden Patiententypen geeignet sein: Früh- oder Neugeborene, Säuglinge, Babys, Kinder, Jugendliche, Erwachsene.

Der Druckmessanschluss kann beispielsweise als eine Komponente einer Steckverbindung zum Verbinden der Nasenkanüle mit einer Atemgasquelle ausgeführt sein.

Mittels der Druckmessleitung ist es möglich, den in der Nase oder im Nasenrachenraum herrschenden Druck getrennt vom Atemgasstrom im Zufuhrschlauch an den Druckmessanschluss zu übertragen. Dazu kann die Druckmessleitung beispielsweise als ein separater Schlauch, ein separates Rohr oder eine Kombination aus mindestens einem separaten Schlauch und mindestens einem separaten Rohr ausgebildet sein.

Ein zweiter Aspekt der Erfindung betrifft ein Beatmungssystem. Das Beatmungssystem umfasst eine Nasenkanüle gemäß Anspruch 7 und einen Zufuhrschlauch zum Verbinden des Schlauchanschlusses der Nasenkanüle mit einer Atemgasquelle, sodass Atemgas von der Atemgasquelle über den Zufuhrschlauch in den Grundkörper der Nasenkanüle strömen kann. Ferner umfasst das Beatmungssystem ein an den Druckmessanschluss der Nasenkanüle angeschlossenes Druckmessgerät zum Umwandeln eines am Druckmessanschluss anliegenden (pneumatischen) Drucks in ein elektrisches Druckmesssignal und eine Steuereinheit zum Steuern mindestens eines Beatmungsparameters der (nasalen) High-Flow-Therapie unter Verwendung des Druckmesssignals.

Die Steuereinheit kann Hard- und/oder Softwaremodule umfassen. Insbesondere kann die Steuereinheit einen Prozessor umfassen, der konfiguriert ist, um ein entsprechendes (computerimplementiertes) Verfahren zum Steuern und/oder Auswerten der Beatmungsparameter auszuführen. Zusätzlich kann die Steuereinheit einen Speicher und/oder eine Datenkommunikationsschnittstelle zur drahtlosen und/oder drahtgebundenen Datenkommunikation mit Peripheriegeräten umfassen. Alternativ kann die Steuereinheit ausschließlich als Hardware, beispielsweise in Form eines ASIC- oder FPGA-Bausteins, implementiert sein.

Der mindestens eine Beatmungsparameter kann beispielsweise mindestens einen der folgenden Parameter umfassen: einen Atemgasdruck, einen Atemgasfluss, ein Atemgasvolumen, eine Atemgaszusammensetzung.

Ein solches Beatmungssystem ermöglicht eine besonders präzise Steuerung der High-Flow-Therapie im Vergleich zu herkömmlichen Beatmungssystemen ohne direkte Messung des Drucks in der Nasenkanüle.

Im Folgenden werden verschiedene Ausführungsformen der Offenbarung beschrieben. Diese Ausführungsformen sind nicht als Beschränkung des Umfangs der Offenbarung zu verstehen.

Gemäß einer Ausführungsform kann mindestens eine der Komponenten des Nasenansatzstücks zumindest teilweise aus Silikon gefertigt sein.

Gemäß einer Ausführungsform können die verschiedenen Komponenten des Nasenansatzstücks aus dem gleichen (Kunststoff-)Material, beispielsweise Silikon, gefertigt sein.

Gemäß einer Ausführungsform kann das Nasenansatzstück einstückig ausgebildet sein. Beispielsweise kann das Nasenansatzstück in einem Spritzgießverfahren gefertigt worden sein.

Gemäß einer Ausführungsform kann die Nasenkanüle so ausgebildet sein, dass das Nasenansatzstück einerseits über das erste Wangenpolster und andererseits über das zweite Wangenpolster am Halteband befestigt werden kann. Anders ausgedrückt kann die Nasenkanüle so ausgebildet sein, dass an jedem Wangenpolster einerseits (unmittelbar) das Halteband und andererseits (unmittelbar) der Grundkörper befestigt werden kann. Die Wangenpolster bieten aufgrund ihrer flächigen Form in der Regel genügend Platz für eine stabile und komfortable Befestigung des Haltebands. Im Vergleich zu den Wangenpolstern ist das Nasenansatzstück in der Regel eher lang und schmal ausgeformt. Die Möglichkeiten für eine unmittelbare Befestigung des Haltebands am Nasenansatzstück sind daher meist beschränkt und mit gewissen Kompromissen in Bezug auf die Stabilität und/oder den Komfort verbunden. Indem die Wangenpolster als tragende Verbindungselemente konzipiert werden, können derartige Beschränkungen bzw. Kompromisse ohne großen zusätzlichen Aufwand vermieden werden.

Gemäß einer Ausführungsform kann die Nasenkanüle so ausgebildet sein, dass mindestens eines oder jedes der Wangenpolster über eine Klettverbindung am Halteband und/oder am Nasenansatzstück befestigt ist oder werden kann. Dies ermöglicht eine komfortable und/oder sichere Befestigung in verschiedenen Positionen mit geringem konstruktivem Aufwand. Eine derartige Verbindung kann zudem sehr oft gelöst werden, ohne dass nennenswerter Verschleiß auftritt. Alternativ oder zusätzlich ist eine Klebeverbindung zum Befestigen des jeweiligen Wangenpolsters am Halteband und/oder am Nasenansatzstück möglich.

Gemäß einer Ausführungsform kann mindestens eines oder jedes der Wangenpolster an einer ersten Seite, die der jeweiligen Wange beim Gebrauch der Nasenkanüle zugewandt ist, einen hautfreundlichen Berührungsabschnitt zum Berühren der jeweiligen Wange aufweisen und an einer der ersten Seite gegenüberliegenden zweiten Seite, die der jeweiligen Wange beim Gebrauch der Nasenkanüle abgewandt ist, einen Befestigungsabschnitt zum Befestigen des Wangenpolsters am Halteband und/oder am Nasenansatzstück aufweisen. Der Befestigungsabschnitt kann einen Haftpart und/oder einen Flauschpart einer Klettverbindung und/oder einen (selbsthaftenden) Klebeabschnitt zum Befestigen des Wangenpolsters am Halteband und/oder am Nasenansatzstück umfassen.

Gemäß einer Ausführungsform kann der Berührungsabschnitt zumindest teilweise aus Silikon sein und/oder eine spezielle Klebefläche zum Kleben des jeweiligen Wangenpolsters auf die jeweilige Wange umfassen. Ein Berührungsabschnitt ohne Klebefläche hat den Vorteil, dass keine Kleberückstände von der Haut entfernt werden müssen.

Gemäß einer Ausführungsform kann mindestens eines oder jedes der Wangenpolster als ein Lagenverbund aus mindestens einer ersten Lage und einer zweiten Lage ausgebildet sein, die sich in ihrem Material und/oder ihrer Größe (beispielsweise ihrer Dicke) und/oder ihrer Form voneinander unterscheiden können. Dabei kann der Berührungsabschnitt ein Abschnitt der ersten Lage und/oder der Befestigungsabschnitt ein Abschnitt der zweiten Lage sein. Die zweite Lage kann als Träger zum Stabilisieren der ersten Lage ausgebildet sein (oder umgekehrt). Beispielsweise kann der Träger aus einem (z. B. besonders luftdurchlässigen) Textilmaterial gefertigt sein.

Gemäß einer Ausführungsform kann das Nasenansatzstück ferner einen vom Grundkörper abstehenden ersten Befestigungsflügel zum Befestigen des ersten Wangenpolsters am Nasenansatzstück und/oder einen vom Grundkörper abstehenden zweiten Befestigungsflügel zum Befestigen des zweiten Wangenpolsters am Nasenansatzstück umfassen. Der erste Befestigungsflügel kann so am Grundkörper befestigt sein, dass er beim Gebrauch der Nasenkanüle über ein erstes Ende des Grundkörpers hin zur ersten Wange hinausragt. Alternativ oder zusätzlich kann der zweite Befestigungsflügel so am Grundkörper befestigt sein, dass er beim Gebrauch der Nasenkanüle über ein zweites Ende des Grundkörpers hin zur zweiten Wange hinausragt. Beispielsweise kann der Befestigungsflügel oder können die Befestigungsflügel jeweils in Längsrichtung des (länglichen) Grundkörpers betrachtet zumindest teilweise über ein Ende des Grundkörpers hinausragen. Alternativ oder zusätzlich kann der Befestigungsflügel oder können die Befestigungsflügel zumindest teilweise seitlich über den Grundkörper hinausragen.

Der Befestigungsflügel kann oder die Befestigungsflügel können jeweils fest oder wiederlösbar, beispielsweise über eine Klett- und/oder Klebeverbindung, mit dem Grundkörper verbunden sein. Der Befestigungsflügel kann oder die Befestigungsflügel können im Vergleich zum Grundkörper besonders flexibel ausgebildet sein, um eine gute Anpassung an die Gesichtskonturen des Patienten zu ermöglichen. Insbesondere kann der oder können die Befestigungsflügel aus einem (z. B. besonders luftdurchlässigen) Textilmaterial gefertigt sein und/oder zumindest abschnittsweise eine flauschige Oberfläche aufweisen. Denkbar ist auch, dass der oder die Befestigungsflügel und der Grundkörper einstückig und/oder aus dem gleichen Material ausgebildet sind.

Gemäß einer Ausführungsform kann die Nasenkanüle so ausgebildet sein, dass zum Befestigen des ersten Wangenpolsters am Nasenansatzstück der Befestigungsabschnitt des ersten Wangenpolsters am ersten Befestigungsflügel befestigt werden kann und/oder zum Befestigen des zweiten Wangenpolsters am Nasenansatzstück der Befestigungsabschnitt des zweiten Wangenpolsters am zweiten Befestigungsflügel befestigt werden kann. Somit kann das jeweilige Wangenpolster sicher und/oder präzise am Nasenansatzstück befestigt werden, ohne dass dadurch gewünschte Eigenschaften des jeweiligen Wangenpolsters wie Flexibilität oder Luftdurchlässigkeit nennenswert beeinträchtigt werden.

Gemäß einer Ausführungsform kann der erste Befestigungsflügel eine durchgehende Öffnung aufweisen, die eine Oberseite des ersten Befestigungsflügels mit einer der Oberseite gegenüberliegenden Unterseite des ersten Befestigungsflügels verbindet. Die Öffnung kann so angeordnet sein, dass der am ersten Befestigungsflügel befestigte Befestigungsabschnitt des ersten Wangenpolsters der Öffnung zumindest teilweise gegenüberliegt, sodass das Halteband an dem der Öffnung gegenüberliegenden Befestigungsabschnitt des ersten Wangenpolsters befestigt werden kann.

Gemäß einer Ausführungsform kann der zweite Befestigungsflügel eine durchgehende Öffnung aufweisen, die eine Oberseite des zweiten Befestigungsflügels mit einer der Oberseite gegenüberliegenden Unterseite des zweiten Befestigungsflügels verbindet. Die Öffnung kann so angeordnet sein, dass der am zweiten Befestigungsflügel befestigte Befestigungsabschnitt des zweiten Wangenpolsters der Öffnung zumindest teilweise gegenüberliegt, sodass das Halteband an dem der Öffnung gegenüberliegenden Befestigungsabschnitt des zweiten Wangenpolsters befestigt werden kann.

Dabei kann der der Öffnung gegenüberliegende Teil des jeweiligen Befestigungsabschnitts zumindest einen Teil des Haftparts und/oder des Flauschparts umfassen. In diesem Fall kann der jeweilige Befestigungsflügel einen mit dem Flauschpart verhakbaren weiteren Haftpart und/oder einen mit dem Haftpart verhakbaren weiteren Flauschpart aufweisen.

Dies ermöglicht eine besonders kompakte Ausführung der Nasenkanüle.

Gemäß einer Ausführungsform kann das Halteband so ausgebildet sein, dass es beim Gebrauch der Nasenkanüle mindestens 50 %, mindestens 70 % oder mindestens 90 % einer Region des Kopfes außerhalb des Gesichts bedeckt. Anders ausgedrückt kann das Halteband im gebrauchsfähigen Zustand mützenartig ausgebildet sein. Somit kann das Halteband den Kopf ähnlich einer Mütze zusätzlich wärmen und/oder schützen.

Gemäß einer Ausführungsform kann das Halteband einen ersten Bandteil zum Befestigen des Haltebands am ersten Wangenpolster und einen vom ersten Bandteil getrennten zweiten Bandteil zum Befestigen des Haltebands am zweiten Wangenpolster umfassen und so ausgebildet sein, dass der erste Bandteil mit dem zweiten Bandteil in verschiedenen Positionen zu dem Halteband verbunden werden kann, um eine Anpassung einer Größe und/oder einer Form des Haltebands an den Kopf zu ermöglichen. Es ist möglich, dass das Halteband mindestens zweiteilig, mindestens dreiteilig oder mindestens vierteilig ausgeführt ist, wobei das Halteband so ausgebildet sein kann, dass die einzelnen Bandteile wiederlösbar miteinander verbunden werden können. Beispielsweise können die einzelnen Bandteile in ihrer Länge und/oder Breite verstellbar sein. Dies ermöglicht eine besonders präzise Anpassung des Haltebands an verschiedene Kopfgrößen und/oder -formen.

Gemäß einer Ausführungsform kann das Halteband so ausgebildet sein, dass der erste Bandteil mit dem zweiten Bandteil über eine Klettverbindung zu dem Halteband verbunden werden kann. Dies ermöglicht ein besonders sicheres und/oder komfortables Anlegen bzw. Abnehmen des Haltebands. Zudem kann eine solche Klettverbindung im Vergleich zu anderen Verbindungstypen wie beispielsweise Riemen- oder Schnallenverbindungen sehr weich und/oder biegsam ausgeführt werden.

Gemäß einer Ausführungsform kann jeder der Bandteile einen Wangenbandabschnitt zum Befestigen des Bandteils am jeweiligen Wangenpolster, einen ersten Kopfbandabschnitt und einen zweiten Kopfbandabschnitt umfassen. In diesem Fall kann das Halteband so ausgebildet sein, dass die ersten Kopfbandabschnitte der verschiedenen Bandteile in verschiedenen Positionen miteinander zu einem ersten Kopfband verbunden werden können, um eine Anpassung einer Größe und/oder einer Form des ersten Kopfbands an den Kopf zu ermöglichen, und die zweiten Kopfbandabschnitte der verschiedenen Bandteile in verschiedenen Positionen miteinander zu einem zweiten Kopfband verbunden werden können, um eine Anpassung einer Größe und/oder einer Form des zweiten Kopfbands an den Kopf zu ermöglichen. Ferner kann das Halteband so ausgebildet sein, dass beim Gebrauch der Nasenkanüle:
- der am ersten Wangenpolster befestigte Wangenbandabschnitt um eine erste Wangenregion, die zumindest einen Teil der ersten Wange umfasst, gespannt ist,
- der am zweiten Wangenpolster befestigte Wangenbandabschnitt um eine von der ersten Wangenregion abweichende zweite Wangenregion, die zumindest einen Teil der zweiten Wange umfasst, gespannt ist,
- das erste Kopfband um eine erste Kopfregion, die zumindest einen Teil einer Scheitelregion des Kopfes umfasst, gespannt ist und
- das zweite Kopfband um eine von der ersten Kopfregion abweichende zweite Kopfregion, die zumindest einen Teil einer Hinterhauptregion und/oder einer Nackenregion des Kopfes umfasst, gespannt ist.

Dies ermöglicht auch bei sehr lebhaften Patienten eine sichere Fixierung des Nasenansatzstücks gegenüber dem Gesicht. Des Weiteren kann der durch das Halteband ausgeübte Druck auf diese Weise besonders gleichmäßig über den Kopf verteilt werden. Somit können unerwünschte Druckstellen auch bei längerem Gebrauch der Nasenkanüle vermieden werden.

Jede der Wangenregionen kann zusätzlich zumindest einen Teil des jeweiligen Ohrs und/oder der jeweiligen Schläfe des Patienten umfassen.

Beispielsweise kann jedes der Kopfbänder in jeder der verschiedenen Positionen, in denen die jeweiligen Kopfbandabschnitte miteinander verbunden werden können, eine andere Länge und/oder Breite haben.

Gemäß einer Ausführungsform kann jeder der Bandteile ferner einen dritten Kopfbandabschnitt umfassen. In diesem Fall kann das Halteband so ausgebildet sein, dass die dritten Kopfbandabschnitte der verschiedenen Bandteile in verschiedenen Positionen miteinander zu einem dritten Kopfband verbunden werden können, um eine Anpassung einer Größe und/oder einer Form des dritten Kopfbands an den Kopf zu ermöglichen. Ferner kann das Halteband so ausgebildet sein, dass beim Gebrauch der Nasenkanüle das dritte Kopfband um eine von der ersten Kopfregion und/oder der zweiten Kopfregion abweichende dritte Kopfregion des Kopfes gespannt ist. Beispielsweise kann die dritte Kopfregion zumindest einen Teil der Nackenregion umfassen.

Das Halteband kann auch mehr als drei solcher Kopfbänder umfassen.

Gemäß einer Ausführungsform kann das Halteband so ausgebildet sein, dass mindestens einer der Kopfbandabschnitte des ersten Bandteils mit dem jeweiligen Kopfbandabschnitt des zweiten Bandteils über eine Klettverbindung zu dem jeweiligen Kopfband verbunden werden kann. Dies ermöglicht ein besonders sicheres und/oder komfortables Anlegen bzw. Abnehmen des jeweiligen Kopfbands. Zudem kann eine solche Klettverbindung im Vergleich zu anderen Verbindungstypen wie beispielsweise Riemen- oder Schnallenverbindungen sehr weich und/oder biegsam ausgeführt werden.

Beispielsweise kann das Halteband so ausgebildet sein, dass benachbarte Kopfbänder beim Gebrauch der Nasenkanüle jeweils durch eine Aussparung, die einen Teil des Kopfes freigibt, voneinander getrennt sind. Dies verbessert die Belüftung im Vergleich zu einer Ausführungsform ohne solche Aussparungen.

Es ist möglich, dass jeder der Bandabschnitte in einem Bereich, in dem der jeweilige Bandabschnitt beim Gebrauch der Nasenkanüle den Kopf berührt, eine Breite und/oder Länge von mindestens 2 cm, vorzugsweise mindestens 4 cm, hat. Auf diese Weise kann vermieden werden, dass die einzelnen Bandabschnitte übermäßigen Druck auf den besonders empfindlichen Kopf ausüben.

Denkbar ist auch, dass jeder der Bandteile einen Grundabschnitt umfasst, von dem die jeweiligen Bandabschnitte fingerartig in verschiedenen Richtungen abgehen.

Gemäß einer Ausführungsform kann die Nasenkanüle ferner einen Steckverbinder zum Verbinden eines freien Endes des an den Schlauchanschluss angeschlossenen Zufuhrschlauchs mit einer Atemgasquelle über eine Steckverbinderaufnahme umfassen. Der Steckverbinder kann so ausgebildet sein, dass er mit der Steckverbinderaufnahme zu einer luftdichten Steckverbindung zusammengesteckt (und bei Bedarf wieder davon getrennt) werden kann. Der Steckverbinder kann - wenn er mit der Steckverbinderaufnahme zusammengesteckt ist - so an und/oder in der Steckverbinderaufnahme gelagert sein, dass der Steckverbinder und die Steckverbinderaufnahme relativ zueinander um eine gemeinsame Drehachse gedreht werden können. Beispielsweise kann die Lagerung so ausgebildet sein, dass der Steckverbinder und die Steckverbinderaufnahme relativ zueinander um mehr als 360 Grad und/oder in beliebigen Drehrichtungen gedreht werden können. Auf diese Weise kann ein unerwünschtes Verdrehen und/oder Knicken von Schläuchen, die an die Steckverbindung angeschlossen sind, vermieden werden.

Gemäß einer Ausführungsform kann die Nasenkanüle ferner den Zufuhrschlauch umfassen, wobei der Zufuhrschlauch einerseits an den Schlauchanschluss und andererseits an den Steckverbinder angeschlossen ist.

Gemäß einer Ausführungsform kann die Nasenkanüle ferner die Steckverbinderaufnahme und/oder einen Verbindungsschlauch zum Verbinden der Steckverbinderaufnahme mit der Atemgasquelle umfassen. Bei dem Verbindungsschlauch kann es sich beispielsweise um einen im Vergleich zum vorstehend beschriebenen Zufuhrschlauch besonders dicken und/oder stabilen (Spiral-)Schlauch handeln.

Gemäß einer Ausführungsform kann der Steckverbinder ein rohrförmiges Innenteil zum Leiten des Atemgases und ein rohrförmiges Außenteil umfassen. Der Außendurchmesser des Innenteils kann um einen vordefinierten Betrag kleiner als der Innendurchmesser des Außenteils sein und das Innenteil und das Außenteil können koaxial angeordnet sein, sodass das Innenteil und das Außenteil einen definierten ringförmigen Spalt zum Aufnehmen zumindest eines Abschnitts der Steckverbinderaufnahme begrenzen. Der Spalt kann so ausgebildet sein, dass der vom Spalt aufgenommene Abschnitt der Steckverbinderaufnahme in Umfangsrichtung des Spalts bewegt werden kann, um eine Drehung des Steckverbinders relativ zur Steckverbinderaufnahme um eine gemeinsame Mittelachse des Innenteils und des Außenteils als die gemeinsame Drehachse zu ermöglichen.

Gemäß einer Ausführungsform kann die Steckverbindung eine genormte Steckverbindung, insbesondere nach DIN EN ISO 5356, sein. In der Norm DIN EN ISO 5356 werden konische Konnektoren für Anästhesie- und Beatmungsgeräte beschrieben. Solche Konnektoren können in Form männlicher und weiblicher Konen (DIN EN ISO 5356-1) oder in Form gewichtstragender Konnektoren mit Schraubgewinde (DIN EN ISO 5356-2) ausgeführt sein.

Gemäß einer Ausführungsform kann der Steckverbinder ein Befestigungselement zum Befestigen des Steckverbinders, beispielsweise an einem Kleidungsstück oder einem Schlauch, aufweisen. Ein solches Befestigungselement kann beispielsweise als eine Klammer und/oder ein Bügel (ähnlich wie bei einem Kugelschreiber) ausgebildet sein.

Gemäß einer Ausführungsform kann die Nasenkanüle ferner einen Schlauchhalter zum Halten des Zufuhrschlauchs in einer definierten Position gegenüber dem Gesicht des Patienten umfassen. Der Schlauchhalter kann beispielsweise so ausgebildet sein, dass er wiederlösbar, beispielsweise durch Klemmen, Kletten oder Kleben, an einem der Wangenpolster und/oder am Halteband befestigt werden kann.

Gemäß einer Ausführungsform kann das Nasenansatzstück ferner einen weiteren Schlauchanschluss zum Anschließen eines weiteren Zufuhrschlauchs an den Grundkörper umfassen, sodass Atemgas aus dem weiteren Zufuhrschlauch in den Grundkörper strömen kann. Der Schlauchanschluss und der weitere Schlauchanschluss können beispielsweise an in Längsrichtung des Grundkörpers einander gegenüberliegenden Enden des (länglichen) Grundkörpers angeordnet sein.

Gemäß einer Ausführungsform kann der Steckverbinder ausgebildet sein, um ferner ein freies Ende des an den weiteren Schlauchanschluss angeschlossenen weiteren Zufuhrschlauchs mit der Atemgasquelle über die Steckverbinderaufnahme zu verbinden.

Anders ausgedrückt kann der Steckverbinder einerseits einen mit der Atemgasquelle verbindbaren Eingang und andererseits einen mit den beiden Zufuhrschläuchen verbindbaren Ausgang aufweisen, wobei der Eingang mit dem Ausgang fluidisch gekoppelt ist. Beispielsweise kann der Steckverbinder zwei separate Ausgänge für die beiden Zufuhrschläuche aufweisen, sodass diese unabhängig voneinander mit dem Steckverbinder verbunden werden können.

Gemäß einer Ausführungsform kann die Nasenkanüle ferner den weiteren Zufuhrschlauch umfassen, wobei der weitere Zufuhrschlauch einerseits an den weiteren Schlauchanschluss und andererseits an den Steckverbinder angeschlossen ist.

Gemäß einer Ausführungsform kann die Nasenkanüle ferner einen Druckmessanschluss zum Anschließen eines Druckmessgeräts (zum Erfassen eines Luftdrucks) an die Nasenkanüle und eine Druckmessleitung umfassen. Die Druckmessleitung kann mit dem Druckmessanschluss fluidisch gekoppelt sein und sich ausgehend vom Druckmessanschluss durch den Grundkörper bis in mindestens einen der Nasenstutzen erstrecken, um eine (Luft-)Druckmessung in der Nase, beispielsweise am Austritt des Nasenstutzens oder der Nasenstutzen, zu ermöglichen. Dies ermöglicht eine sehr genaue oropharyngeale Druckmessung während der High-Flow-Therapie (siehe weiter oben). Beispielsweise kann sich ein Abschnitt der Druckmessleitung ausgehend vom Druckmessanschluss durch den Zufuhrschlauch bis in den Grundkörper erstrecken. Anders ausgedrückt kann die Druckmessleitung zumindest teilweise in den Zufuhrschlauch integriert sein. Dies kann die Handhabung der Nasenkanüle vereinfachen.

Gemäß einer Ausführungsform kann der Druckmessanschluss in ein Gehäuse des Steckverbinders, beispielweise in das vorgenannte Innen- und/oder Außenteil, integriert sein. Anders ausgedrückt kann der Steckverbinder so ausgebildet sein, dass er gleichzeitig an die Atemgasquelle und das Druckmessgerät angeschlossen werden kann. Dies hat den Vorteil, dass ein separater Druckmessanschluss entfallen kann, was Platz spart und die Handhabung der Nasenkanüle vereinfachen kann.

### Kurze Beschreibung der Zeichnungen

Im Folgenden werden Ausführungsformen der Offenbarung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Beatmungssystem gemäß einer Ausführungsform der Offenbarung.
Fig. 2 zeigt einen Querschnitt durch eine Steckverbindung aus Fig. 1.
Fig. 3 zeigt ein Halteband einer Nasenkanüle gemäß einer Ausführungsform der Offenbarung mit zwei Kopfbändern.
Fig. 4 zeigt ein Halteband einer Nasenkanüle gemäß einer Ausführungsform der Offenbarung mit drei Kopfbändern.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Offenbarung

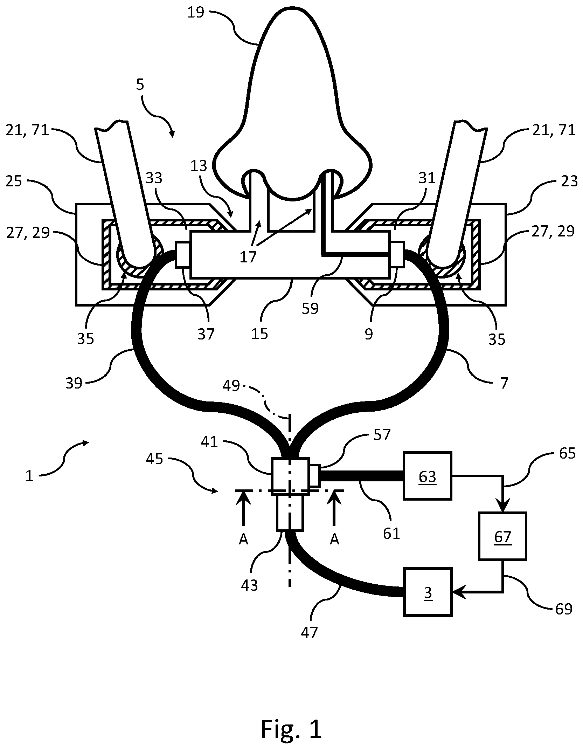

Fig. 1 zeigt ein Beatmungssystem 1 zum Beatmen eines Patienten, hier eines Früh- oder Neugeborenen, eines Säuglings oder eines Babys, im Rahmen einer nasalen High-Flow-Therapie. Das Beatmungssystem 1 umfasst eine Atemgasquelle 3 zum Bereitstellen eines Atemgases, beispielsweise Sauerstoff oder Druckluft mit erhöhter Sauerstoffkonzentration, eine Nasenkanüle 5 und einen Zufuhrschlauch 7, über den ein Schlauchanschluss 9 der Nasenkanüle 3 mit der Atemgasquelle 3 verbunden ist, sodass Atemgas von der Atemgasquelle 3 über den Zufuhrschlauch 7 in die Nasenkanüle 5 strömen kann. Die Nasenkanüle 5 umfasst ein Nasenansatzstück 13 mit einem hohlen, beispielsweise rohr- oder zylinderförmigen Grundkörper 15 und zwei vom Grundkörper 15 abstehenden Nasenstutzen 17 zum Einführen in die Nase 19 des Patienten. Das Nasenansatzstück 13 ist so ausgebildet, dass das Atemgas aus dem Zufuhrschlauch 7 über den Schlauchanschluss 9 in den Grundkörper 15 und von dort über die Nasenstutzen 17 in die Nasenlöcher der Nase 19 strömen kann.

Die Nasenkanüle 5 kann je nach Ausführungsform auch für mindestens einen der folgenden Patiententypen geeignet sein: Kinder, Jugendliche, Erwachsene.

Es ist möglich, dass mindestens eine der Komponenten des Nasenansatzstücks 13 zumindest teilweise aus Silikon gefertigt ist und/oder das gesamte Nasenansatzstück 13 einstückig ausgebildet ist. Denkbar ist auch, dass die verschiedenen Komponenten des Nasenansatzstücks 13 aus dem gleichen Material, beispielsweise Silikon oder einem anderen geeigneten Kunststoff, gefertigt sind.

Des Weiteren umfasst die Nasenkanüle 5 ein um den Kopf des Patienten spannbares Halteband 21 zum Fixieren des Nasenansatzstücks 13 in einer definierten Position gegenüber dem Gesicht des Patienten, beispielsweise durch Aufbringen einer entsprechenden zusätzlichen Haltekraft auf das Nasenansatzstück 13. Das Halteband 21 kann als ein vom Zufuhrschlauch 7 getrenntes längliches Element, beispielsweise als ein Gummi- und/oder Stoffband oder als eine Anordnung aus mehreren solcher Bänder, ausgeführt sein.

Ferner umfasst die Nasenkanüle 5 beispielhaft ein erstes Wangenpolster 23 und ein zweites Wangenpolster 25 zum zusätzlichen Abstützen und/oder Fixieren des gegenüber dem Gesicht fixierten Nasenansatzstücks 13 an den beiden Wangen. Die Wangenpolster 23, 25 können jeweils als ein flächiges, besonders biegsames Element aus einem luftdurchlässigen und/oder flüssigkeitsabsorbierenden Material, beispielsweise einem besonders hautfreundlichen und/oder besonders weichen Textilmaterial, ausgeführt sein und die Haut des Patienten beim Gebrauch der Nasenkanüle 5 unmittelbar flächig berühren. Dadurch kann der Anpressdruck, der durch das um den Kopf gespannte Halteband 21 erzeugt wird, über eine möglichst große Fläche des Gesichts, insbesondere der Wangen, gleichmäßig verteilt werden. Somit können unerwünschte Druckstellen im Gesicht vermieden werden.

Die Nasenkanüle 5 kann so ausgebildet sein, dass das Nasenansatzstück 13 einerseits über das erste Wangenpolster 23 und andererseits über das zweite Wangenpolster 25 am Halteband 21 befestigt werden kann, beispielsweise über eine oder mehrere Klett- und/oder Klebeverbindungen.

In diesem Beispiel hat jedes der Wangenpolster 23, 25 an einer ersten Seite, die der jeweiligen Wange beim Gebrauch der Nasenkanüle 5 zugewandt ist, einen hautfreundlichen Berührungsabschnitt zum Berühren der jeweiligen Wange und an einer der ersten Seite gegenüberliegenden zweiten Seite, die der jeweiligen Wange beim Gebrauch der Nasenkanüle 5 abgewandt ist, einen Befestigungsabschnitt 27. Der Befestigungsabschnitt 27 umfasst hier einen Haft- und/oder Flauschpart 29 einer Klettverbindung zum Befestigen des Wangenpolsters 23 bzw. 25 am Halteband 21 und/oder am Nasenansatzstück 13. Alternativ oder zusätzlich kann der Befestigungsabschnitt 27 zu diesem Zweck einen (selbsthaftenden) Klebeabschnitt umfassen.

Der Berührungsabschnitt kann zumindest teilweise aus Silikon sein und/oder eine spezielle Klebefläche zum Kleben des jeweiligen Wangenpolsters 23 bzw. 25 auf die jeweilige Wange umfassen.

Es ist möglich, dass jedes der Wangenpolster 23, 25 als ein Lagenverbund aus mindestens einer ersten Lage und einer zweiten Lage ausgebildet ist, wobei sich die Lagen in ihrem Material und/oder ihrer Größe (beispielsweise ihrer Dicke) und/oder ihrer Form voneinander unterscheiden können. Dabei kann der Berührungsabschnitt ein Abschnitt der ersten Lage und/oder der Befestigungsabschnitt 27 ein Abschnitt der zweiten Lage sein. Die zweite Lage kann als Träger zum Stabilisieren der ersten Lage ausgebildet sein (oder umgekehrt). Beispielsweise kann der Träger aus einem besonders luftdurchlässigen Textilmaterial gefertigt sein. Möglich sind aber auch andere Trägermaterialien.

Zusätzlich umfasst das Nasenansatzstück 13 einen vom Grundkörper 15 abstehenden ersten Befestigungsflügel 31 zum Befestigen des ersten Wangenpolsters 23 am Nasenansatzstück 13 und einen vom Grundkörper 15 abstehenden zweiten Befestigungsflügel 33 zum Befestigen des zweiten Wangenpolsters 25 am Nasenansatzstück 13. Dabei kann der Befestigungsabschnitt 27 des ersten Wangenpolsters 23 am ersten Befestigungsflügel 31 bzw. der Befestigungsabschnitt 27 des zweiten Wangenpolsters 25 am zweiten Befestigungsflügel 33 per Klettverschluss befestigt sein.

Die Befestigungsflügel 31, 33 können jeweils fest oder wiederlösbar, beispielsweise über eine Klett- und/oder Klebeverbindung, mit dem Grundkörper 15 verbunden sein. Es ist möglich, dass die Befestigungsflügel 31, 33 im Vergleich zum Grundkörper 15 besonders flexibel ausgebildet sind, sodass sie sich gut an die Gesichtskonturen des Patienten anpassen können. Insbesondere können die Befestigungsflügel 31, 33 aus einem beispielsweise besonders luftdurchlässigen Textilmaterial gefertigt sein und/oder zumindest abschnittsweise eine flauschige Oberfläche aufweisen. Denkbar ist auch, dass die Befestigungsflügel 31, 33 und der Grundkörper 15 einstückig und/oder aus dem gleichen Material ausgebildet sind.

Es ist möglich, dass jeder der Befestigungsflügel 31, 33 eine durchgehende Öffnung 35 aufweist, die eine Oberseite des Befestigungsflügels 31 bzw. 33 mit dessen jeweiliger Unterseite verbindet. Die Öffnung 35 kann jeweils so angeordnet sein, dass sie dem jeweiligen Befestigungsabschnitt 27 des am jeweiligen Befestigungsflügel 31 bzw. 33 befestigten Wangenpolsters 23 bzw. 25 zumindest teilweise gegenüberliegt. Somit kann das Halteband 21 an den den Öffnungen 35 gegenüberliegenden Teilen der Befestigungsabschnitte 27 befestigt werden, hier beispielhaft per Klettverschluss.

In diesem Beispiel umfasst der Grundkörper 15 zusätzlich einen weiteren Schlauchanschluss 37, der über einen weiteren Zufuhrschlauch 39 mit der Atemgasquelle 3 verbunden sein kann, sodass das Atemgas zusätzlich aus dem weiteren Zufuhrschlauch 39 in den Grundkörper 15 und von dort über die Nasenstutzen 17 in die Nase 19 strömen kann.

Die freien Enden der beiden Zufuhrschläuche 7, 39 können jeweils an einen Steckverbinder 41 angeschlossen sein, der mit einer entsprechenden Steckverbinderaufnahme 43 zusammengesteckt werden kann, um eine luftdichte Steckverbindung 45 zu bilden, die durch Auseinanderziehen des Steckverbinders 41 und der Steckverbinderaufnahme 43 mit einem gewissen Kraftaufwand wieder gelöst werden kann. Die Steckverbinderaufnahme 43 kann über einen Verbindungsschlauch 47 mit der Atemgasquelle 3 verbunden sein, sodass das Atemgas über den Verbindungsschlauch 47 und die Steckverbindung 45 in die beiden Zufuhrschläuche 7, 39 strömen kann.

Die Steckverbindung 45 kann optional als eine Drehverbindung ausgebildet sein, die eine gezielte Verdrehung des Steckverbinders 41 relativ zur Steckverbinderaufnahme 43 um eine gemeinsame Drehachse 49 ermöglicht, beispielsweise um mehr als 360 Grad und/oder in beliebigen Drehrichtungen.

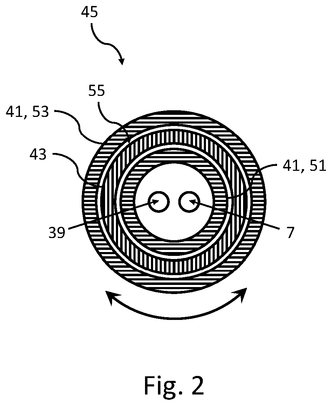

Wie aus Fig. 2 ersichtlich, wo ein Querschnitt durch die Steckverbindung 41 aus Fig. 1 entlang einer Schnittlinie A-A gezeigt wird, kann der Steckverbinder 41 zu diesem Zweck beispielsweise mit einem rohrförmigen oder hülsenartigen Innenteil 51 zum Leiten des Atemgases und einem rohrförmigen oder hülsenartigen Außenteil 53 ausgeführt sein. Dabei kann der Außendurchmesser des Innenteils 51 um einen definierten Betrag kleiner als der Innendurchmesser des Außenteils 53 sein und das Innenteil 51 und das Außenteil 53 können derart koaxial angeordnet sein, dass sie einen definierten ringförmigen Spalt 55 zum Lagern der Steckverbinderaufnahme 43 begrenzen. Der Spalt 55 kann so ausgebildet sein, dass er zumindest einen Abschnitt der Steckverbinderaufnahme 43 aufnehmen kann und der vom Spalt 55 aufgenommene Abschnitt der Steckverbinderaufnahme 43 relativ zum Innenteil 51 bzw. zum Außenteil 53 mit geringem Kraftaufwand in beide Drehrichtungen gedreht werden kann. In diesem Beispiel stimmt die Drehachse 49 mit einer gemeinsamen Mittelachse des Innenteils 51 und des Außenteils 53 überein.

Die freien Enden der beiden Zufuhrschläuche 7, 39 können beispielsweise an einen entsprechenden Anschluss oder entsprechende (separate) Anschlüsse des Innenteils 51 angeschlossen sein.

Insbesondere kann die Steckverbindung 45 als eine genormte Steckverbindung, beispielweise nach DIN EN ISO 5356-1 oder 5356-2, ausgeführt sein.

Wie in Fig. 1 gezeigt, kann die Nasenkanüle 5 zusätzlich einen Druckmessanschluss 57 und eine mit dem Druckmessanschluss 57 verbundene Druckmessleitung 59 umfassen. Die Druckmessleitung 59 kann sich ausgehend vom Druckmessanschluss 57 bis in mindestens einen der beiden Nasenstutzen 17 erstrecken. Dies ermöglicht eine oropharyngeale Druckmessung während der nasalen High-Flow-Therapie.

In diesem Beispiel ist der Druckmessanschluss 57 in ein Gehäuse des Steckverbinders 41 integriert. Dabei erstreckt sich die Druckmessleitung 59 durch den Zufuhrschlauch 7 und den Grundkörper 15 bis zum offenen Ende des rechten der beiden Nasenstutzen 17.

Der Druckmessanschluss 57 kann über einen weiteren Verbindungsschlauch 61 mit einem Druckmessgerät 63 des Beatmungssystems 1 verbunden sein. Das Druckmessgerät 63 kann ausgebildet sein, um einen am Druckmessanschluss 57 anliegenden pneumatischen Druck in ein entsprechendes elektrisches Druckmesssignal 65 umzuwandeln.

Das Druckmesssignal 65 kann von einer entsprechend konfigurierten Steuereinheit 67 des Beatmungssystems 1 verwendet werden, um mindestens einen Beatmungsparameter der High-Flow-Therapie zu steuern. Ein solcher Beatmungsparameter kann je nach Ausführungsform ein Atemgasdruck, ein Atemgasfluss, ein Atemgasvolumen oder eine Atemgaszusammensetzung sein. Beispielsweise kann die Steuereinheit 67 konfiguriert sein, um abhängig vom derart gemessenen oropharyngealen Druck ein Steuersignal 69 zum Steuern mindestens eines Aktors der Atemgasquelle 3, z. B. eines Gebläses oder eines Ventils, zu erzeugen.

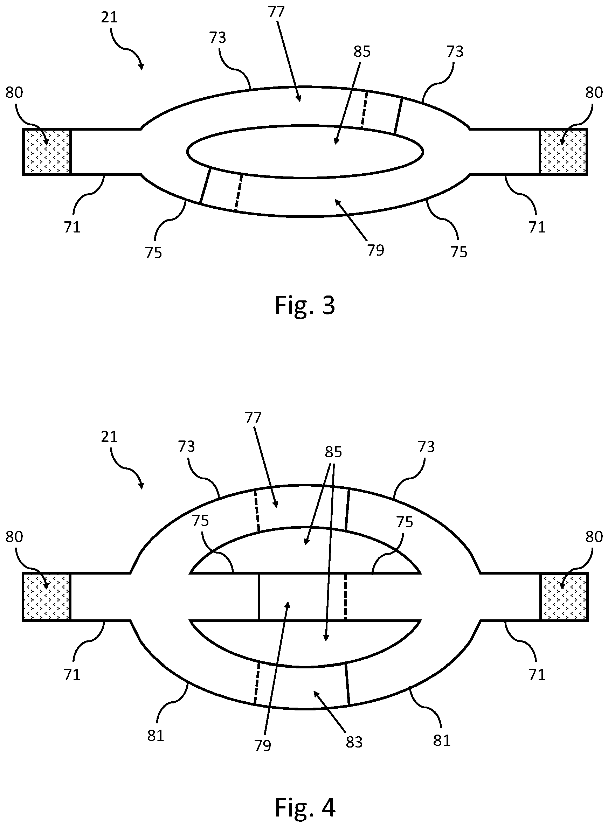

Wie aus Fig. 3 und Fig. 4 ersichtlich, kann das Halteband 21 einen ersten Bandteil zum Befestigen des Haltebands 21 am ersten Wangenpolster 23 und einen vom ersten Bandteil getrennten zweiten Bandteil zum Befestigen des Haltebands 21 am zweiten Wangenpolster 25 umfassen. Die beiden Bandteile können - beispielsweise über einen oder mehrere Klettverschlüsse - in verschiedenen Positionen miteinander verbunden werden, um ein an die jeweilige Kopfgröße bzw. -form angepasstes Halteband 21 zu bilden.

Fig. 3 zeigt eine Ausführungsform, bei der jeder der Bandteile einen Wangenbandabschnitt 71 zum Befestigen des Bandteils am jeweiligen Wangenpolster 23 bzw. 25, einen ersten Kopfbandabschnitt 73 und einen zweiten Kopfbandabschnitt 75 umfasst. Die ersten Kopfbandabschnitte 73 der verschiedenen Bandteile können in verschiedenen Positionen miteinander verbunden werden, um ein an die jeweilige Kopfgröße bzw. -form angepasstes erstes Kopfband 77 zu bilden. Ebenso können die zweiten Kopfbandabschnitte 75 der verschiedenen Bandteile in verschiedenen Positionen miteinander verbunden werden, um ein an die jeweilige Kopfgröße bzw. -form angepasstes zweites Kopfband 79 zu bilden.

Das Halteband 21 kann so ausgebildet sein, dass beim Gebrauch der Nasenkanüle 5 der am ersten Wangenpolster 23 befestigte Wangenbandabschnitt 71 um eine erste Wangenregion, die zumindest einen Teil der ersten Wange umfasst, gespannt ist, der am zweiten Wangenpolster 25 befestigte Wangenbandabschnitt 71 um eine von der ersten Wangenregion abweichende zweite Wangenregion, die zumindest einen Teil der zweiten Wange umfasst, gespannt ist, das erste Kopfband 77 um eine erste Kopfregion, die zumindest einen Teil einer Scheitelregion des Kopfes umfasst, gespannt ist und das zweite Kopfband 79 um eine von der ersten Kopfregion abweichende zweite Kopfregion, die zumindest einen Teil einer Hinterhauptregion und/oder einer Nackenregion des Kopfes umfasst, gespannt ist.

Beispielsweise können die Wangenbandabschnitte 71 an ihren freien Enden jeweils einen weiteren Haft- und/oder Flauschpart 80 aufweisen, der mit dem Haft- bzw. Flauschpart 29 des jeweiligen Befestigungsabschnitts 27 verbindbar ist, um die jeweilige Klettverbindung zu bilden.

Jede der Wangenregionen kann zusätzlich zumindest einen Teil des jeweiligen Ohrs und/oder der jeweiligen Schläfe des Patienten umfassen.

Fig. 4 zeigt eine Ausführungsform, bei der jeder der Bandteile zusätzlich einen dritten Kopfbandabschnitt 81 umfasst. Analog zu den vorstehend beschriebenen Kopfbandabschnitten 73, 75 können die dritten Kopfbandabschnitte 81 der verschiedenen Bandteile in verschiedenen Positionen miteinander verbunden werden, um ein an die jeweilige Kopfgröße bzw. -form angepasstes drittes Kopfband 83 zu bilden, das beim Gebrauch der Nasenkanüle 5 um eine von der ersten Kopfregion und/oder der zweiten Kopfregion abweichende dritte Kopfregion des Kopfes gespannt ist. Beispielsweise kann die dritte Kopfregion zumindest einen Teil der Nackenregion umfassen.

Das Halteband 21 kann auch mehr als drei solcher Kopfbänder umfassen.

Beispielsweise kann jedes der Kopfbänder in jeder der verschiedenen Positionen, in denen die jeweiligen Kopfbandabschnitte miteinander verbunden werden können, eine andere Länge und/oder Breite haben.

Es ist möglich, dass jeder der Bandabschnitte in einem Bereich, in dem der jeweilige Bandabschnitt beim Gebrauch der Nasenkanüle 5 den Kopf berührt, eine Breite und/oder Länge von mindestens 2 cm, vorzugsweise mindestens 4 cm, hat. Auf diese Weise kann vermieden werden, dass die einzelnen Bandabschnitte übermäßigen Druck auf den besonders empfindlichen Kopf ausüben.

Das Halteband 21 kann zudem mützenartig ausgebildet sein, sodass es beim Gebrauch der Nasenkanüle 5 mindestens 50 %, mindestens 70 % oder mindestens 90 % einer Region des Kopfes außerhalb des Gesichts bedeckt.

Es ist möglich, dass benachbarte Kopfbänder beim Gebrauch der Nasenkanüle 5 jeweils durch eine Aussparung 85, die einen Teil des Kopfes freigibt, voneinander getrennt sind. Dies verbessert die Belüftung im Vergleich zu einer Ausführungsform ohne solche Aussparungen.

Die jeweiligen Kopfbandabschnitte können beispielsweise per Klettverschluss miteinander verbindbar sein.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungssystem
- 3: Atemgasquelle
- 5: Nasenkanüle
- 7: Zufuhrschlauch
- 9: Schlauchanschluss
- 13: Nasenansatzstück
- 15: Grundkörper
- 17: Nasenstutzen
- 19: Nase
- 21: Halteband
- 23: erstes Wangenpolster
- 25: zweites Wangenpolster
- 27: Befestigungsabschnitt
- 29: Haft- und/oder Flauschpart
- 31: erster Befestigungsflügel
- 33: zweiter Befestigungsflügel
- 35: Öffnung
- 37: weiterer Schlauchanschluss
- 39: weiterer Zufuhrschlauch
- 41: Steckverbinder
- 43: Steckverbinderaufnahme
- 45: Steckverbindung
- 47: Verbindungsschlauch
- 49: Drehachse, Mittelachse
- 51: Innenteil
- 53: Außenteil
- 55: Spalt
- 57: Druckmessanschluss
- 59: Druckmessleitung
- 61: weiterer Verbindungsschlauch
- 63: Druckmessgerät
- 65: Druckmesssignal
- 67: Steuereinheit
- 69: Steuersignal
- 71: Wangenbandabschnitt
- 73: erster Kopfbandabschnitt
- 75: zweiter Kopfbandabschnitt
- 77: erstes Kopfband
- 79: zweites Kopfband
- 80: weiterer Haft- und/oder Flauschpart
- 81: dritter Kopfbandabschnitt
- 83: drittes Kopfband
- 85: Aussparung

## Patentansprüche

1. Nasenkanüle (5) zum Versorgen eines Patienten mit Atemgas im Rahmen einer High-Flow-Therapie, wobei der Patient ein Früh- oder Neugeborenes, ein Säugling oder ein Baby ist, wobei die Nasenkanüle (5) umfasst:
ein Nasenansatzstück (13), das einen hohlen Grundkörper (15), einen Schlauchanschluss (9) zum Anschließen eines Zufuhrschlauchs (7) an den Grundkörper (15), sodass Atemgas aus dem Zufuhrschlauch (7) in den Grundkörper (15) strömen kann, und zwei vom Grundkörper (15) abstehende Nasenstutzen (17) zum Leiten des Atemgases aus dem Grundkörper (15) in die Nase (19) des Patienten umfasst;
ein erstes Wangenpolster (23) zum Abstützen und/oder Fixieren des Nasenansatzstücks (13) an einer ersten Wange des Patienten;
ein zweites Wangenpolster (25) zum Abstützen und/oder Fixieren des Nasenansatzstücks (13) an einer zweiten Wange des Patienten;
ein um den Kopf des Patienten spannbares Halteband (21) zum Aufbringen einer zusätzlichen Haltekraft auf das Nasenansatzstück (13) und/oder die beiden Wangenpolster (23, 25);
wobei jedes der Wangenpolster (23, 25) an einer ersten Seite, die der jeweiligen Wange beim Gebrauch der Nasenkanüle (5) zugewandt ist, einen hautfreundlichen Berührungsabschnitt zum Berühren der jeweiligen Wange aufweist und an einer der ersten Seite gegenüberliegenden zweiten Seite einen Befestigungsabschnitt (27) zum Befestigen des Wangenpolsters (23, 25) am Halteband (21) und am Nasenansatzstück (13) aufweist;
wobei das Nasenansatzstück (13) ferner umfasst:
einen vom Grundkörper (15) abstehenden ersten Befestigungsflügel (31) zum Befestigen des ersten Wangenpolsters (23) am Nasenansatzstück (13);
einen vom Grundkörper (15) abstehenden zweiten Befestigungsflügel (33) zum Befestigen des zweiten Wangenpolsters (25) am Nasenansatzstück (13);
wobei zum Befestigen des ersten Wangenpolsters (23) am Nasenansatzstück (13) der Befestigungsabschnitt (27) des ersten Wangenpolsters (23) am ersten Befestigungsflügel (31) befestigt ist und zum Befestigen des zweiten Wangenpolsters (25) am Nasenansatzstück (13) der Befestigungsabschnitt (27) des zweiten Wangenpolsters (25) am zweiten Befestigungsflügel (33) befestigt ist;
wobei der erste Befestigungsflügel (31) eine durchgehende erste Öffnung (35) aufweist, die eine Oberseite des ersten Befestigungsflügels (31) mit einer der Oberseite gegenüberliegenden Unterseite des ersten Befestigungsflügels (31) verbindet und so angeordnet ist, dass der am ersten Befestigungsflügel (31) befestigte Befestigungsabschnitt (27) des ersten Wangenpolsters (23) der ersten Öffnung (35) zumindest teilweise gegenüberliegt, wobei das Halteband (21) an dem der ersten Öffnung (35) gegenüberliegenden Befestigungsabschnitt (27) des ersten Wangenpolsters (23) befestigt ist, wobei der zweite Befestigungsflügel (33) eine durchgehende zweite Öffnung (35) aufweist, die eine Oberseite des zweiten Befestigungsflügels (33) mit einer der Oberseite gegenüberliegenden Unterseite des zweiten Befestigungsflügels (33) verbindet und so angeordnet ist, dass der am zweiten Befestigungsflügel (33) befestigte Befestigungsabschnitt (27) des zweiten Wangenpolsters (25) der zweiten Öffnung (35) zumindest teilweise gegenüberliegt, wobei das Halteband (21) an dem der zweiten Öffnung (35) gegenüberliegenden Befestigungsabschnitt (27) des zweiten Wangenpolsters (25) befestigt ist.

2. Nasenkanüle (5) nach einem der vorhergehenden Ansprüche,
wobei jeder Befestigungsabschnitt (27) einen Haftpart (29) und/oder einen Flauschpart (29) einer Klettverbindung zum Befestigen des jeweiligen Wangenpolsters (23, 25) am Halteband (21) und am Nasenansatzstück (13) umfasst.

3. Nasenkanüle (5) nach einem der vorhergehenden Ansprüche,
wobei das Halteband (21) einen ersten Bandteil zum Befestigen des Haltebands (21) am ersten Wangenpolster (23) und einen vom ersten Bandteil getrennten zweiten Bandteil zum Befestigen des Haltebands (21) am zweiten Wangenpolster (25) umfasst und so ausgebildet ist, dass der erste Bandteil mit dem zweiten Bandteil in verschiedenen Positionen zu dem Halteband (21) verbunden werden kann, um das Halteband (21) in seiner Größe und/oder Form an den Kopf anzupassen.

4. Nasenkanüle (5) nach Anspruch 3,
wobei jeder der Bandteile einen Wangenbandabschnitt (71) zum Befestigen des Bandteils am jeweiligen Wangenpolster (23, 25), einen ersten Kopfbandabschnitt (73) und einen zweiten Kopfbandabschnitt (75) umfasst;
wobei die ersten Kopfbandabschnitte (73) der verschiedenen Bandteile in verschiedenen Positionen miteinander zu einem ersten Kopfband (77) verbindbar sind, um das erste Kopfband (77) in seiner Größe und/oder Form an den Kopf anzupassen;
wobei die zweiten Kopfbandabschnitte (75) der verschiedenen Bandteile in verschiedenen Positionen miteinander zu einem zweiten Kopfband (79) verbindbar sind, um das zweite Kopfband (79) in seiner Größe und/oder Form an den Kopf anzupassen;
wobei das Halteband (21) so ausgebildet ist, dass beim Gebrauch der Nasenkanüle (5) der am ersten Wangenpolster (23) befestigte Wangenbandabschnitt (71) um eine erste Wangenregion, die zumindest einen Teil der ersten Wange umfasst, gespannt ist, der am zweiten Wangenpolster (25) befestigte Wangenbandabschnitt (71) um eine von der ersten Wangenregion abweichende zweite Wangenregion, die zumindest einen Teil der zweiten Wange umfasst, gespannt ist, das erste Kopfband (77) um eine erste Kopfregion, die zumindest einen Teil einer Scheitelregion des Kopfes umfasst, gespannt ist und das zweite Kopfband (79) um eine von der ersten Kopfregion abweichende zweite Kopfregion, die zumindest einen Teil einer Hinterhauptregion und/oder einer Nackenregion des Kopfes umfasst, gespannt ist.

5. Nasenkanüle (5) nach Anspruch 4,
wobei jeder der Bandteile ferner einen dritten Kopfbandabschnitt (81) umfasst;
wobei die dritten Kopfbandabschnitte (81) der verschiedenen Bandteile in verschiedenen Positionen miteinander zu einem dritten Kopfband (83) verbindbar sind, um das dritte Kopfband (83) in seiner Größe und/oder Form an den Kopf anzupassen;
wobei das Halteband (21) so ausgebildet ist, dass beim Gebrauch der Nasenkanüle (5) das dritte Kopfband (83) um eine von der ersten Kopfregion und/oder der zweiten Kopfregion abweichende dritte Kopfregion des Kopfes gespannt ist.

6. Nasenkanüle (5) nach Anspruch 4 oder 5,
wobei mindestens einer der Kopfbandabschnitte (73, 75, 81) des ersten Bandteils mit dem jeweiligen Kopfbandabschnitt (73, 75, 81) des zweiten Bandteils über eine Klettverbindung zu dem jeweiligen Kopfband (77, 79, 83) verbindbar ist.

7. Nasenkanüle (5) nach einem der vorhergehenden Ansprüche, ferner umfassend:
einen Druckmessanschluss (57) zum Anschließen eines Druckmessgeräts (63) an die Nasenkanüle (5);
eine Druckmessleitung (59), die mit dem Druckmessanschluss (57) fluidisch gekoppelt ist und sich ausgehend vom Druckmessanschluss (57) durch den Grundkörper (15) bis in mindestens einen der Nasenstutzen (17) erstreckt, um eine Druckmessung in der Nase (19) zu ermöglichen.

8. Nasenkanüle (5) nach einem der vorhergehenden Ansprüche, ferner umfassend:
einen Steckverbinder (41) zum Verbinden eines freien Endes des an den Schlauchanschluss (9) angeschlossenen Zufuhrschlauchs (7) mit einer Atemgasquelle (3) über eine Steckverbinderaufnahme (43), wobei der Steckverbinder (41) so ausgebildet ist, dass der Steckverbinder (41) mit der Steckverbinderaufnahme (43) zu einer luftdichten Steckverbindung (45) zusammengesteckt werden kann, wobei der Steckverbinder (41) - wenn er mit der Steckverbinderaufnahme (43) zusammengesteckt ist - so an und/oder in der Steckverbinderaufnahme (43) gelagert ist, dass der Steckverbinder (41) und die Steckverbinderaufnahme (43) relativ zueinander um eine gemeinsame Drehachse (49) gedreht werden können.

9. Nasenkanüle (5) nach Anspruch 8 rückbezogen auf Anspruch 7,
wobei der Druckmessanschluss (57) in ein Gehäuse des Steckverbinders (41) integriert ist.

10. Beatmungssystem (1), umfassend:
eine Nasenkanüle (5) nach Anspruch 7;
einen Zufuhrschlauch (7) zum Verbinden des Schlauchanschlusses (9) der Nasenkanüle (5) mit einer Atemgasquelle (3), sodass Atemgas von der Atemgasquelle (3) über den Zufuhrschlauch (7) in den Grundkörper (15) der Nasenkanüle (5) strömen kann;
ein an den Druckmessanschluss (57) der Nasenkanüle (5) angeschlossenes Druckmessgerät (63) zum Umwandeln eines am Druckmessanschluss (57) anliegenden Drucks in ein elektrisches Druckmesssignal (65);
eine Steuereinheit (67) zum Steuern mindestens eines Beatmungsparameters der High-Flow-Therapie unter Verwendung des Druckmesssignals (65).

## Claims

1. A nasal cannula (5) for supplying a patient with breathing gas in the context of high-flow therapy, wherein the patient is a baby, a premature baby, or a newborn, wherein the nasal cannula (5) comprises:
a nose piece (13) comprising a hollow main body (15), a hose connection (9) for connecting a supply tube (7) to the main body (15) so that breathing gas can flow from the supply tube (7) into the main body (15), and two nose prongs (17) projecting from the main body (15) for conducting the breathing gas from the main body (15) into the patient's nose (19);
a first cheek cushion (23) for supporting and/or securing the nose piece (13) on a first cheek of the patient;
a second cheek cushion (25) for supporting and/or securing the nose piece (13) on a second cheek of the patient;
a support strap (21) that can be tensioned around the patient's head to apply an additional holding force to the nose piece (13) and/or the two cheek cushions (23, 25);
wherein each of the cheek cushions (23, 25), on a first side facing the corresponding cheek during use of the nasal cannula (5), has a skin-friendly contact portion for touching the corresponding cheek and, on a second side opposite to the first side, a fastening portion (27) for fastening the cheek cushion (23, 25) to the support strap (21) and to the nose piece (13);
wherein the nose piece (13) also comprises:
a first fastening wing (31) projecting from the main body (15) for fastening the first cheek cushion (23) to the nose piece (13);
a second fastening wing (33) projecting from the main body (15) for fastening the second cheek cushion (25) to the nose piece (13);
wherein, in order to fasten the first cheek cushion (23) to the nose piece (13), the fastening portion (27) of the first cheek cushion (23) is fastened to the first fastening wing (31) and, in order to fasten the second cheek cushion (25) to the nose piece (13), the fastening portion (27) of the second cheek cushion (25) is fastened to the second fastening wing (33);
wherein the first fastening wing (31) has a first through-opening (35), which connects the upper side of the first fastening wing (31) to an underside, which is opposite to the upper side, of the first fastening wing (31) and is arranged such that the fastening portion (27), which is fastened to the first fastening wing (31), of the first cheek cushion (23) lies at least partly opposite to the first opening (35), wherein the support strap (21) is fastened to the fastening portion (27), which is opposite to the first opening (35), of the first cheek cushion (23), wherein the second fastening wing (33) has a second through-opening (35), which connects an upper side of the second fastening wing (33) to an underside, which is opposite to the upper side, of the second fastening wing (33) and is arranged such that the fastening portion (27), which is fastened on the second fastening wing (33), of the second cheek cushion (25) lies at least partly opposite to the second opening (35), wherein the support strap (21) is fastened to the fastening portion (27), which is opposite to the second opening (35), of the second cheek cushion (25).

2. The nasal cannula (5) according to one of the preceding claims,
wherein each fastening portion (27) has a hook part (29) and/or a loop part (29) of a hook-and-loop fastener for fastening the corresponding cheek cushion (23, 25) to the support strap (21) and/or to the nose piece (13).

3. The nasal cannula (5) according to one of the preceding claims,
wherein the support strap (21) comprises a first strap part for fastening the support strap (21) to the first cheek cushion (23) and a second strap part, which is separate from the first strap part, for fastening the support strap (21) to the second cheek cushion (25) and is designed such that the first strap part can be connected to the second strap part in various positions to form the support strap (21) in order to adapt the size and shape of the support strap (21) to the head.

4. The nasal cannula (5) according to claim 3,
wherein each of the strap parts comprises a cheek strap portion (71) for fastening the strap part to the corresponding cheek cushion (23, 25), a first head strap portion (73), and a second head strap portion (75);
wherein the first head strap portions (73) of the various strap parts can be connected to each other in various positions to form a first head strap (77) in order to adapt the size and/or shape of the first head strap (77) to the head;
wherein the second head strap portions (75) of the various strap parts can be connected to each other in various positions to form a second head strap (79) in order to adapt the size and/or shape of the second head strap (79) to the head;
wherein the support strap (21) is designed such that, during use of the nasal cannula (5), the cheek strap portion (71) fastened to the first cheek cushion (23) is tensioned around a first cheek region that comprises at least a part of the first cheek, the cheek strap portion (71) fastened to the second cheek cushion (25) is tensioned around a second cheek region that differs from the first cheek region and comprises at least a part of the second cheek, the first head strap (77) is tensioned around a first head region that comprises at least a part of the crown region of the head, and the second head strap (79) is tensioned around a second head region that differs from the first head region and comprises at least a part of an occipital region and/or a neck region of the head.

5. The nasal cannula (5) according to claim 4,
wherein each of the strap parts also comprises a third head strap portion (81);
wherein the third head strap portions (81) of the various strap parts can be connected to each other in various positions to form a third head strap (83) in order to adapt the size and/or shape of the third head strap (83) to the head;
wherein the support strap (21) is designed such that, during use of the nasal cannula (5), the third head strap (83) is tensioned around a third head region of the head that differs from the first head region and/or the second head region.

6. The nasal cannula (5) according to claim 4 or 5,
wherein at least one of the head strap portions (73, 75, 81) of the first strap part can be connected to the corresponding head strap portion (73, 75, 81) of the second strap part by a hook-and-loop fastener to form the corresponding head strap (77, 79, 83).

7. The nasal cannula (5) according to one of the preceding claims, further comprising:
a pressure measurement connection (57) for connecting a pressure gauge (63) to the nasal cannula (5);
a pressure measurement line (59) that is fluidically coupled to the pressure measurement connection (57) and extends, starting from the pressure measurement connection (57), through the main body (15) and into at least one of the nose prongs (17) to enable pressure to be measured in the nose (19).

8. The nasal cannula (5) according to one of the preceding claims, further comprising:
a plug connector (41) for connecting a free end of the supply tube (7), which is connected to the tube connection (9), to a breathing gas source (3) via a plug connector receptacle (43), wherein the plug connector (41) is designed such that the plug connector (41) can be plugged into the plug connector receptacle (43) to form an air-tight plug connection (45), wherein the plug connector (41) - when it is plugged into the plug connector receptacle (43) - is mounted on and/or in the plug connector receptacle (43) such that the plug connector (41) and the plug connector receptacle (43) can be rotated relative to each other about a common axis of rotation (49).

9. The nasal cannula (5) according to claim 8, when dependent on claim 7,
wherein the pressure measurement connection (57) is integrated into a housing of the plug connector (41).

10. A ventilation system (1), comprising:
a nasal cannula (5) according to claim 7;
a supply tube (7) for connecting the tube connection (9) of the nasal cannula (5) to a breathing gas source (3) so that breathing gas can flow from the breathing gas source (3) via the supply tube (7) and into the main body (15) of the nasal cannula (5);
a pressure gauge (63) connected to the pressure measurement connection (57) of the nasal cannula (5) for converting a pressure applied to the pressure measurement connection (57) into an electrical pressure measurement signal (65);
a control unit (67) for controlling at least one ventilation parameter of the high-flow therapy using the pressure measurement signal (65).

## Revendications

1. Canule nasale (5) destinée à fournir du gaz respiratoire à un patient dans le cadre d'un traitement à haut débit, le patient étant un prématuré ou un nouveau-né, un nourrisson ou un bébé, la canule nasale (5) comportant :
un embout nasal (13) comportant un corps de base creux (15), un raccord de tube (9) pour le raccordement d'un tube d'alimentation (7) au corps de base (15), de sorte que du gaz respiratoire peut s'écouler du tube d'alimentation (7) vers le corps de base (15), et deux tubulures nasales (17) faisant saillie à partir du corps de base (15) pour guider le gaz respiratoire du corps de base (15) vers le nez (19) du patient ;
un premier coussinet de joue (23) pour l'appui et/ou la fixation de l'embout nasal (13) sur une première joue du patient ;
un deuxième coussinet de joue (25) pour l'appui et/ou la fixation de l'embout nasal (13) sur une deuxième joue du patient ;
une bande de maintien (21) apte à être serrée autour de la tête du patient pour appliquer une force de maintien supplémentaire à l'embout nasal (13) et/ou aux deux coussinets de joue (23, 25) ;
dans laquelle chacun des coussinets de joue (23, 25) présente, sur un premier côté tourné vers la joue respective pendant l'utilisation de la canule nasale (5), une section hypoallergénique destinée à être en contact avec la joue respective, et présente, sur un deuxième côté opposé au premier côté, une section de fixation (27) destinée à fixer le coussinet de joue (23, 25) à la bande de maintien (21) et à l'embout nasal (13) ;
dans laquelle l'embout nasal (13) comporte en outre :
une première ailette de fixation (31) faisant saillie à partir du corps de base (15) pour fixer le premier coussinet de joue (23) à l'embout nasal (13) ;
une deuxième ailette de fixation (33) faisant saillie à partir du corps de base (15) pour fixer le deuxième coussinet de joue (25) à l'embout nasal (13) ;
dans laquelle, pour fixer le premier coussinet de joue (23) à l'embout nasal (13), la section de fixation (27) du premier coussinet de joue (23) est fixée à la première ailette de fixation (31) et, pour fixer le deuxième coussinet de joue (25) à l'embout nasal (13), la section de fixation (27) du deuxième coussinet de joue (25) est fixée à la deuxième ailette de fixation (33) ;
dans laquelle la première ailette de fixation (31) présente une première ouverture traversante (35), laquelle relie un côté supérieur de la première ailette de fixation (31) à un côté inférieur de la première ailette de fixation (31) opposé au côté supérieur, et laquelle est disposée de telle façon que la section de fixation (27) du premier coussinet de joue (23) fixée à la première ailette de fixation (31) est au moins partiellement opposée à la première ouverture (35), dans laquelle la bande de maintien (21) est fixée à la section de fixation (27) du premier coussinet de joue (23) opposée à la première ouverture (35), dans laquelle la deuxième ailette de fixation (33) présente une deuxième ouverture traversante (35), laquelle relie un côté supérieur de la deuxième ailette de fixation (33) à un côté inférieur de la deuxième ailette de fixation (33) opposé au côté supérieur, et laquelle est disposée de telle façon que la section de fixation (27) du deuxième coussinet de joue (25) fixée à la deuxième ailette de fixation (33) est au moins partiellement opposée à la deuxième ouverture (35), dans laquelle la bande de maintien (21) est fixée à la section de fixation (27) du deuxième coussinet de joue (25) opposée à la deuxième ouverture (35).

2. Canule nasale (5) selon l'une des revendications précédentes,
dans laquelle chaque section de fixation (27) comporte une partie adhésive (29) et/ou une partie de velcro (29) d'une liaison auto-agrippante destinée à fixer le coussinet de joue (23, 25) respectif à la bande de maintien (21) et à l'embout nasal (13).

3. Canule nasale (5) selon l'une des revendications précédentes,
dans laquelle la bande de maintien (21) comporte une première partie de bande destinée à fixer la bande de maintien (21) au premier coussinet de joue (23) et une deuxième partie de bande distincte de la première partie de bande, destinée à fixer la bande de maintien (21) au deuxième coussinet de joue (25), et est conçue de telle façon que la première partie de bande peut être reliée à la deuxième partie de bande dans différentes positions pour former la bande de maintien (21), afin d'adapter la bande de maintien (21) à la tête quant à sa taille et/ou à sa forme.

4. Canule nasale (5) selon la revendication 3,
dans laquelle chacune des parties de bande comporte une section de bande de joue (71) destinée à fixer la partie de bande au coussinet de joue (23, 25) respectif, une première section de bande de tête (73) et une deuxième section de bande de tête (75) ;
dans laquelle les premières sections de bande de tête (73) des différentes parties de bande peuvent être reliées dans différentes positions les unes aux autres en une première bande de tête (77) pour adapter la première bande de tête (77) à la tête quant à sa taille et/ou à sa forme ;
dans laquelle les deuxième sections de bande de tête (75) des différentes parties de bande peuvent être reliées dans différentes positions les unes aux autres en une deuxième bande de tête (79) pour adapter la deuxième bande de tête (79) à la tête quant à sa taille et/ou à sa forme ;
dans laquelle la bande de maintien (21) est conçue de telle façon que lors de l'utilisation de la canule nasale (5), la section de bande de joue (71) fixée au premier coussinet de joue (23) est tendue autour d'une première région de joue, laquelle comporte au moins une partie de la première joue, la section de bande de joue (71) fixée au deuxième coussinet de joue (25) est tendue autour d'une deuxième région de joue différente de la première région de joue, laquelle comporte au moins une partie de la deuxième joue, la première bande de tête (77) est tendue autour d'une première région de tête, laquelle comporte au moins une partie d'une région de sommet de la tête, et la deuxième bande de tête (79) est tendue autour d'une deuxième région de tête différente de la première région de tête, laquelle comporte au moins une partie d'une région occipitale principale et/ou d'une région de nuque de la tête.

5. Canule nasale (5) selon la revendication 4,
dans laquelle chacune des parties de bande comporte en outre une troisième section de bande de tête (81) ;
dans laquelle les troisièmes sections de bande de tête (81) des différentes parties de bande peuvent être reliées dans différentes positions les unes aux autres en une troisième bande de tête (83) pour adapter la troisième bande de tête (83) à la tête quant à sa taille et/ou à sa forme ;
dans laquelle la bande de maintien (21) est conçue de telle façon que lors de l'utilisation de la canule nasale (5), la troisième bande de tête (83) est tendue autour d'une troisième région de tête différente de la première région de tête et/ou de la deuxième région de tête.

6. Canule nasale (5) selon la revendication 4 ou 5,
dans laquelle l'une au moins des sections de bande de tête (73, 75, 81) de la première partie de bande peut être reliée à la section de bande de tête (73, 75, 81) respective de la deuxième partie de bande par une liaison auto-agrippante pour former la bande de tête (77, 79, 83) respective.

7. Canule nasale (5) selon l'une des revendications précédentes, comportant en outre :
un raccord de mesure de pression (57) destiné à raccorder un appareil de mesure de pression (63) à la canule nasale (5) ;
une ligne de mesure de pression (59) accouplée fluidiquement au raccord de mesure de pression (57) et s'étendant à travers le corps de base (15) au moins jusque dans l'une des tubulures nasales (17) à partir du raccord de mesure de pression (57) pour permettre de mesurer la pression dans le nez (19).

8. Canule nasale (5) selon l'une des revendications précédentes, comportant en outre :
un connecteur enfichable (41) destiné à connecter une extrémité libre du tube d'alimentation (7) raccordé au raccord de tube (9) à une source de gaz respiratoire (3) par le biais d'un logement de connecteur enfichable (43), dans laquelle le connecteur enfichable (41) est conçu de telle façon que le connecteur enfichable (41) peut être assemblé avec le logement de connecteur enfichable (43) de manière à former une connexion enfichable hermétique (45), dans laquelle le connecteur enfichable (41) - lorsqu'il est assemblé avec le logement de connecteur enfichable (43) - est monté de telle façon sur et/ou dans le logement de connecteur enfichable (43) que le connecteur enfichable (41) et le logement de connecteur enfichable (43) peuvent être tournés l'un par rapport à l'autre autour d'un axe de rotation commun (49).

9. Canule nasale (5) selon la revendication 8 lorsqu'elle dépend de la revendication 7,
dans laquelle le raccord de mesure de pression (57) est intégré dans un boîtier du connecteur enfichable (41).

10. Système respiratoire (1), comportant :
une canule nasale (5) selon la revendication 7 ;
un tube d'alimentation (7) destiné à relier le raccord de tube (9) de la canule nasale (5) à une source de gaz respiratoire (3), de manière à ce que du gaz respiratoire puisse s'écouler à partir de la source de gaz respiratoire (3) vers le corps de base (15) de la canule nasale (5) par le biais du tube d'alimentation (7) ;
un appareil de mesure de pression (63) raccordé au raccord de mesure de pression (57) de la canule nasale (5) pour convertir une pression appliquée au raccord de mesure de pression (57) en un signal de mesure de pression électrique (65) ;
une unité de commande (67) destinée à commander au moins un paramètre respiratoire du traitement à haut débit à l'aide du signal de mesure de pression (65).
